# EUROPEAN PATENT APPLICATION

(11) **EP 2 801 396 A1**
(43) Date of publication of application: **12.11.2014**
(21) Application number: 13167242.0
(22) Date of filing: 10.05.2013
(51) Int. Cl.: B01D 1/06, B01D 3/34, B01J 19/02, C07C 273/04, F28F 19/00

(54) **Use of duplex stainless steel in an ammonia-stripping of urea plants**

(71) Applicant: CASALE SA, 6900 Lugano (CH)
(72) Inventor: Sscotto, Andrea, 6932 Breganzona (CH); Zardi, Federico, 6932 Breganzona (CH)
(74) Representative: Zardi, Marco

(57) **Abstract**

In an ammonia stripping or self-stripping plant for the synthesis of urea, it is disclosed that a shell-and-tube stripper has a bundle of tubes made of a duplex stainless steel 29Cr-6.5Ni-2Mo-N according to UNS S32906 or 27Cr-7.6Ni-1Mo-2.3W-N according to UNS S32808. A revamping of urea plants is also disclosed, including the revamping of ammonia stripping plants, self-stripping plants and conventional total recycle plants, wherein said duplex stainless steel is used for the tubes of the stripper.

## Description

### Field of the invention

The present invention refers to the making or the revamping of ammonia-stripping and self-stripping urea plants.

### Prior Art

An overview of the urea synthesis processes and related plants can be found in the Ullmann's Encyclopedia of Industrial Chemistry, Wiley-VCH Verlag, vol. A27.

The stripping processes were introduced in the 1960s and now dominate the production of urea worldwide. In a stripping process, the synthesis solution leaving the reactor is subjected to heating at a high pressure, which is substantially the same pressure of the reactor. As a consequence, the ammonium carbamate decomposes into ammonia and carbon dioxide in the liquid phase, and part of the liberated ammonia and carbon dioxide passes from the liquid phase to the gas phase. The gas phase collected from the stripper, containing the nonconverted ammonia and carbon dioxide, is condensed and recycled to the reactor.

The ammonia-stripping process uses ammonia as a stripping agent, to promote the above process. The self-stripping process uses no stripping agent, the stripping of the solution being achieved only by supplying of heat.

The ammonia-stripping process and the self-stripping process are well known and are also collectively denoted by the term of Snamprogetti process, after the name of their developer. The self-stripping process is also termed thermal stripping process, since heat is responsible for the stripping of the urea solution. A different stripping process is the CO2-stripping process, where gaseous carbon dioxide is used as a stripping agent. The Stamicarbon process and the ACES process are examples of known CO2-stripping processes.

The invention concerns the Snamprogetti process and related plant. In the present description and claims, any reference to a self-stripping process or plant shall be equally read as a reference to the ammonia-stripping process or plant, and vice-versa.

A self-stripping urea plant comprises basically a high-pressure synthesis loop, a medium-pressure section and a low-pressure recovery section. The high-pressure loop typically comprises a synthesis reactor, a steam-heated stripper, and a horizontal kettle condenser which operate substantially at the same pressure, usually around 140 to 160 bar. The urea aqueous solution leaving the reactor, and comprising ammonia and unconverted ammonium carbamate, is stripped obtaining a vapour phase containing ammonia and CO2, which is condensed in the high-pressure condenser and recycled to said reactor. The following medium- and low-pressure sections decompose unreacted carbamate and recycle ammonia from the urea aqueous solution leaving the high-pressure section.

The synthesis loop provides a combination of high pressure, high temperature and presence of corrosive solutions. The ammonium carbamate, in particular, is considered the most aggressive against steel. Accordingly, the choice of suitable materials is a challenge, and the tubes of the stripper are one of the most critical components, since they operate under high temperature and pressure, high concentration of carbamate, and low oxygen.

The ammonia-stripping and the self-stripping plants adopted titanium stripper tubes for a long time. To overcome problems of erosion of the internal part of titanium tubes, bimetallic tubes were introduced, consisting of an external tube made of austenitic stainless steel and an internal tube made of zirconium. Further development of this concept lead to full zirconium tubes or bimetallic titanium-zirconium (Ti-Zr) tubes. In this respect, EP-A-1577632 discloses tubes made of titanium or a titanium alloy coated with a layer of zirconium or a zirconium alloy, suitable for stripper tubes of a Snamprogetti urea plant.

In contrast, the CO2 stripping processes have made use of special stainless steels, including highly alloyed stainless steel and duplex stainless steels. Said duplex steels are distinguished by a two-phase structure showing both ferrite and austenite, and have generally a high chromium content.

To summarize, the prior art has prompted so far the use of high-grade steel for stripper tubes of CO2-stripping plants, and more sophisticated solutions (titanium tubes, zirconium or bimetallic tubes) for the stripper tubes of ammonia-or self-stripping plants.

Since the Snamprogetti plants account for a large portion of the world urea production capacity, there is an incentive to provide an effective way to improve their performance, in particular for revamping the existing plants. The invention faces this problem.

### Disclosure of the invention

The applicant has surprisingly found that certain duplex stainless steels can be successfully employed also in the self-stripping and ammonia stripping plants for the making of the tubes of the stripper. According to the invention, one of the following duplex stainless steels is used:
A) the Safurex® steel, namely 29Cr-6.5Ni-2Mo-N, which is also designated by ASME Code 2295-3 and by UNS S32906,
   or:
B) the DP28W™ steel, namely 27Cr-7.6Ni-1Mo-2.3W-N, which is also designated by ASME Code Case 2496-1 and by UNS S32808.

The above designation of the suitable materials is given according to known standard, which are familiar to a skilled person, that is: the DIN standard nomenclature, the ASME code and the UNS (Unified Numbering System) designation.

A duplex stainless steel according to said option A), that is the Safurex® steel, has preferably the following composition (% by mass):
C: max. 0.05,
Si: max. 0.8
Mn: 0.3 - 4.0
Cr: 28 - 35,
Ni: 3 - 10,
Mo: 1.0 - 4.0
N: 0.2 - 0.6,
Cu: max. 1.0
W: max. 2.0
S: max. 0.01
Ce: 0 - 0.2.

Preferably the ferrite content is 30-70% by volume and more preferably 30-55%.

More preferably, said steel according to option A) contains (% by weight): max. 0.02 C, max. 0.5 Si, Cr 29 to 33, Mo 1.0 to 2.0, N 0.36 to 0.55, Mn 0.3 to 1.0.

A duplex stainless steel according to said option B), that is the DP28W™ steel, has preferably the following composition (% by mass):
C: 0.03 or less,
Si: 0.5 or less,
Mn: 2 or less,
P: 0.04 or less,
S: 0.003 or less,
Cr: 26 or more, but less than 28,
Ni: 6 - 10,
Mo: 0.2 - 1.7,
W: more than 2, but no more than 3,
N: more than 0.3, but no more than 0.4,
the balance being Fe and impurities, wherein the content of Cu as an impurity is not more than 0.3%.

More preferably, said steel according to option B) comprises: 27 to 27.9% chromium (Cr); 7% to 8.2% nickel (Ni); 0.8% to 1.2% molybdenum (Mo); 2% to 2.5% tungsten (W); 0.3 to 0.4% nitrogen (N).

All the above percentages are in mass.

As mentioned above, the use of duplex stainless steel in the field of urea plants has been proposed in the relevant prior art and literature only in combination with the CO2-stripping process, such as the above mentioned Stamicarbon or ACES processes.

It should be noted that the tubes of the stripper of a Snamprogetti urea plant operate at a higher temperature compared to the stripping tubes of a CO2-stripping plant. Typically, the stripper of an ammonia- or self-stripping plant operates with an outlet temperature of around 205 °C or above (for example 200-210 °C), while the stripper of a CO2-stripping plant operates with an outlet temperature around 160 °C. Moreover the CO2 fed to the bottom of the stripper is containing the 02, which is the passivation agent for the corrosion protection of the steel surface. So far, due to the above differences, the duplex stainless steels have been deemed inappropriate for use in the stripper of ammonia-stripping and self-stripping urea plants.

In contrast with the above prejudice, the applicant has found that certain duplex stainless steels, according to the above chemical requirements, can be used for the making of the stripping tubes of ammonia-stripping and self-stripping urea plants.

In accordance with the above, a first aspect of the invention is a shell-and-tube stripper for use in an ammonia stripping or self-stripping plant for the synthesis of urea, wherein said stripper comprises a shell and a bundle of tubes, and is arranged to provide the stripping of a carbamate solution fed to said tubes by means of heat and optionally by means of ammonia as a stripping medium, and wherein the tubes of the stripper are made of said duplex stainless steel.

In use, the tube bundle of said stripper is fed with the reactor effluent (urea solution) flowing inside the tubes. The heat for the stripping process comes, for example, from hot steam which is fed to the shell side, thus heating the tube bundle.

Said stripper is preferably a vertical stripper of the falling-film type. In this case, the urea solution is fed in such a way to form a liquid film flowing downward inside the tubes, while the stripped gaseous phase, comprising ammonia and carbon dioxide, flows counter-currently through the central portion of tubes and is collected at the top of the stripper. Hot steam flowing outside the tubes supplies the heat necessary to decompose the carbamate contained in the solution.

It is particularly preferred to feed a stream containing oxygen (02) in the bottom of the stripper, to provide a passivation agent for protection for the tubes. This can be accomplished, for example, with a dedicated stream of air or more preferably by means of separation of gas/liquid at the top of the reactor. This second embodiment can be used when passivation oxygen is fed to the reactor. A gaseous effluent recovered from top of the reactor comprises unreacted carbon dioxide and ammonia together with some passivation oxygen. Hence, said effluent can be used to protect the stripper.

Another aspect of the invention is a plant for the synthesis of urea which operates according to the ammonia stripping or thermal stripping process. The plant comprises a high-pressure synthesis loop including a synthesis reactor, a shell-and-tube stripper, and a condenser, and is **characterized in that** the stripper comprises tubes made of a duplex stainless steel according to the above options.

A further aspect of the invention concerns the revamping of the existing ammonia stripping or self-stripping urea plants, using a duplex stainless steel according to the above mentioned options, as a material for the tubes of the stripper. For example, a new stripper with tubes made of one of the above mentioned steels may be installed in lieu of an old conventional stripper (with tubes made of a conventional steel, or with titanium tubes or bimetallic tubes). When appropriate, the shell of the stripper could be maintained, replacing just the old tubes with the new duplex steel tubes.

Another application of the invention is the revamping of a conventional total recycle loop into a modern self stripping process with utilization of a duplex steel, according to the above options, in the stripper. The term of total-recycle denotes the old plants without a stripping, where all nonconverted carbon dioxide was recycled as an aqueous solution.

A method for revamping a non-stripping total-recycle urea plant, for example, includes the provision of a high-pressure shell-and-tube stripper, which operates substantially at the pressure of the existing reactor, and the provision of a condenser to form a high-pressure loop. Accordingly, a traditional total-recycle plant is converted into a more efficient stripping plant. The new stripper has tubes made of a duplex stainless steel according to the above options.

A further aspect of the invention is a process for the synthesis of urea according to the self-stripping or ammonia stripping process, including a step of stripping of a reactor effluent in a shell-and-tube stripper, wherein the tubes of said stripper are made of a duplex stainless steel according to the above options.

## Claims

1. A shell-and-tube stripper, for use in an ammonia stripping plant or self-stripping plant for the synthesis of urea, said stripper comprising a shell and a bundle of tubes, and being arranged to provide the stripping of a carbamate solution fed to said tubes by means of heat and optionally by means of ammonia as a stripping medium, said stripper being **characterized in that** the tubes are made of a duplex stainless steel which is one of the following:
A) the Safurex® steel 29Cr-6.5Ni-2Mo-N, which is also designated by ASME Code 2295-3 and by UNS S32906,
or:
B) the DP28W™ steel 27Cr-7.6Ni-1 Mo-2.3W-N, which is also designated by ASME Code Case 2496-1 and by UNS S32808.

2. A stripper according to claim 1, for operation at a temperature of around 205 °C and a pressure of 140 to 160 bar.

3. A stripper according to any of the previous claims, said stripper being of falling-film type.

4. A plant for the synthesis of urea according to the ammonia stripping or thermal stripping process, comprising a high-pressure synthesis loop which includes a synthesis reactor, a shell-and-tube stripper, and a condenser, **characterized in that** said stripper is in accordance with any of claims 1 to 5.

5. A plant according to claim 4, comprising a feed line arranged to feed a stream containing oxygen at the bottom of the stripper.

6. A plant according to claim 5 said feed line of a stream containing oxygen being a dedicated air feed line directed to the bottom of the stripper.

7. A plant according to claim 6, said feed line of a stream containing oxygen being a reactor effluent taken from a gas/liquid separator at the top of said synthesis reactor of the loop.

8. Use of a duplex stainless steel according to option A) or option B) of claim 1, for the making of the tube bundle of a stripper of an ammonia stripping urea plant or thermal stripping urea plant.

9. A method for revamping an ammonia stripping or thermal stripping urea plant, the method being **characterized by** the use of a duplex stainless steel for the provision of the tubes of a shell-and-tube stripper of said plant, said steel being in accordance with option A) or option B) of claim 1.

10. A method according to claim 9, **characterized in that** the original tubes of an existing stripper are replaced with new tubes made of a duplex stainless steel.

11. A method for revamping a non-stripping total-recycle urea plant, the method being **characterized by** the provision of a high-pressure shell-and-tube stripper, said stripper including tubes made of a duplex stainless steel, and said steel being in accordance with option A) or option B) of claim 1.

12. A process for the synthesis of urea according to the self-stripping or ammonia stripping process, including a step of stripping of a reactor effluent in a shell-and-tube stripper, wherein the tubes of said stripper are made of a duplex stainless steel according to option A) or B) of claim 1.
